# EUROPEAN PATENT SPECIFICATION

(11) **EP 1 807 509 B1**
(45) Date of publication and mention of the grant of the patent: **18.11.2009**
(21) Application number: 05776026.6
(22) Date of filing: 26.08.2005
(51) Int. Cl.: C12N 5/08, A61K 35/12, C12N 5/06

(54) **MULTIPOTENT STEM CELLS ISOLATED FROM UMBILICAL CORD BLOOD AND THE CELLULAR THERAPEUTIC AGENT COMPRISING THE SAME FOR TREATING ISCHEMIC DISEASE**
MULTIPOTENTE BLUTSTAMMZELLEN AUS DER NABELSCHNUR UND ZELLBEHANDLUNGSMITTEL DAMIT ZUR BEHANDLUNG DER ISCHÄMISCHEN KRANKHEIT
CELLULES SOUCHES MULTIPOTENTES ISOLEES DU SANG DE CORDON OMBILICAL ET AGENT THERAPEUTIQUE CELLULAIRE CONTENANT CES CELLULES EN VUE DE TRAITER UNE MALADIE ISCHEMIQUE

(43) Date of publication of application: 18.07.2007
(73) Proprietor: SEOUL NATIONAL UNIVERSITY INDUSTRY FOUNDATION, Gwanak-gu Seoul 151-050 (KR)
(72) Inventor: KANG, Kyung Sun, Seocho-gu, Seoul 137-935 (KR)
(74) Representative: Hiebl, Inge Elisabeth
(86) International application number: PCT/KR2005/002834
(87) International publication number: WO 2007/024036

(56) References cited:
- WO-A-2007/064090
- WO-A1-2005/045010
- WO-A1-2005/073366
- WO-A2-03/068937
- WO-A2-2005/003333
- KR-A- 20030 069 115
- US-A1- 2002 164 794
- POJDA Z ET AL: "NONHEMATOPOIETIC STEM CELLS OF FETAL ORIGIN--HOW MUCH OF TODAY'S ENTHUSIASM WITH PASS THE TIME TEST?" FOLIA HISTOCHEMICA ET CYTOBIOLOGICA, VESALIUS UNIVERSITY MEDICAL PUBLISHER, KRAKOW, PL, vol. 43, no. 4, 1 January 2005 (2005-01-01), pages 209-212, XP009075834 ISSN: 0239-8508
- GANG E J ET AL: "In vitro mesengenic potential of human umbilical cord blood-derived mesenchymal stem cells" BIOCHEMICAL AND BIOPHYSICAL RESEARCH COMMUNICATIONS, ACADEMIC PRESS INC. ORLANDO, FL, US, vol. 321, no. 1, 13 August 2004 (2004-08-13), pages 102-108, XP004521404 ISSN: 0006-291X

## Description

### TECHNICAL FIELD

The present invention relates to multipotent stem cells, adult stem cells isolated by culturing umbilical cord blood in a medium containing human serum or plasma, and a cellular therapeutic agent for ischemic necrosis resulting from occlusive arterial disease, which contains the multipotent stem cells as active ingredients.

### BACKGROUND ART

With the aging of society, the case of blood circulatory disorder caused by occlusive arterial disease has been increasing. Typical diseases of this blood circulatory disorder include myocardial infarction, cerebral infraction, Buerger's disease which is occlusive peripheral vascular disease where blood vessels contract or become narrow due to inflammation or vascular wall changes in small arteries or veins in hands or feet, mainly below knees, so that blood vessels are occluded entirely, as well as occlusive arterial disease caused by microvascular or macrovascular disorders resulting from diabetic complication.

Myocardial infarction is a disease where a portion of coronary artery supplying oxygen and nutrients to the heart is occluded to stop blood circulation so that a cardiac wall (i.e. myocardium) at that portion decays. Cerebral infarctions include thrombotic cerebral infarction where blood vessels become narrow or are occluded due to atherosclerosis to cause inhibition of blood flow and lack of oxygen so that cerebral tissue is killed, and embolic cerebral infarction where a piece (embolus) detached from the thrombus of an artery with atherosclerosis snags on vascular wall to block arteries during its flowing through cerebral blood vessels so that cerebral tissue downstream thereof is killed.

It is inferred that Buerger's disease is resulted from the complex action of various factors, although its direct cause was not found. Age, sex, family-genetic factors, autoimmunity, professions, smoking, and the like are considered as secondary causative factors. Also, because patients with Buerger's disease have high blood fibrinogen levels, the constriction of peripheral blood vessels resulting from the overcoagulation of blood, the overaggregation of platelets or the oversensitiveness of the sympathetic nerve is presumed to be a causative factor.

Gangrene of extremities, which is one of the most terrible symptoms in diabetic complication, occurs in severe diabetics and is a hazardous symptom where hand or foot ends decay in black. Although its exact cause is not known, it is inferred to result from symptoms, such as external wounds, burns and purulence. The gangrene of extremities is a disease which frequently occurs to over 50 years-old patients and shows symptoms of inflammation, blisters, ulcers, fever, and the like, and in severe cases, leads to the cutting of hands and feet or death.

This occlusive arterial disease occurs in higher frequency to Korean than it dose to white men and assumed to account for about 15% of total peripheral arterial diseases, although its authentic statistical data in Korea are not yet sufficient (Laohapensang, K. et al., Eur. J. Vasc. Endovasc. Surg., 28(4):418, 2004). The occlusive arterial disease invades small or middle-sized arteries and veins in the distal upper and lower limbs, and the stagnation of arterial circulation at the end of limbs mostly causes peripheral circulatory disorders to break down the tissue of microvascular system, resulting in ischemic disease. In this ischemic necrosis, due to the reduction of arterial blood flow pressure and the stagnation of capillary blood flow, leucocytes and platelets are activated and vascular endothelium gets damages, resulting in local hypoxia and metabolic changes.

Therapies for occlusive arterial disease include arterial bypass surgery where artificial blood vessels are used to make new blood vessels between the upper and lower portions of occluded arteries such that blood can flow well through the arteries, as well as drug therapy of administering vasodilators, and a therapeutic method of anesthetizing the sympathetic nerve of the lumbar so as to enlarge small blood vessels in limbs. Recently, studies for the development of a therapeutic method with the use of vascular endothelial growth factor 165 (VEGF 165) (Kim, H.J. et al., Exp. Mol. Med., 36(4):336, 2004) and the development of a therapeutic method by autologous hematopoietic cell transplantation (Miyamoto, M. et al., Cell Transplant., 13 (4):429, 2004) are being conducted.

The exact cause of ischemic necrosis is not yet found and the disease has a characteristic that it continues to progress, therefore, it is in a stage where a sure method of treatment is not yet established. An ultimate purpose of treating ischemic necrosis caused by occlusive arterial diseases, such as Buerger's disease, is to make arterial blood flow freely.

Meanwhile, the term "stem cells" refers to cells having not only self-replicaiton ability but also the ability to differentiate into at least two cells, and can be divided into totipotent stem cells, pluripotent stem cells, and multipotent stem cells.

Totipotent stem cells are cells having totipotent properties capable of developing into one perfect individual, and these properties are possessed by cells up to the 8-cell stage after the fertilization of an oocyte and a sperm. When these cells are isolated and transplanted into the uterus, they can develop into one perfect individual.

Pluripotent stem cells, which are cells capable of developing into various cells and tissues derived from the ectodermal, mesodermal and endodermal layers, are derived from an inner cell mass located inside of blastocysts generated 4-5 days after fertilization. These cells are called "embryonic stem cells" and can differentiate into various other tissue cells but not form new living organisms.

Multipotent stem cells, which are stem cells capable of differentiating into only cells specific to tissues and organs containing these cells, are involved not only in the growth and development of various tissues and organs in the fetal, neonatal and adult periods but also in the maintenance of homeostasis of adult tissue and the function of inducing regeneration upon tissue damage. Tissue-specific multipotent cells are collectively called "adult stem cells".

Umbilical cord blood-derived adult stem cells known up to now include mesenchymal stem cells capable of differentiating into osteocytes or skeleton muscles (Lee, O.K. et al., Blood, 103:1669, 2004; Gang, E.J. et al., BBRC, 321:102, 2004; Gang, E.J. et al., Stem Cell, 22:617, 2004), heart stem cells capable of differentiating into heart cells (US 2004/0126879) and endothelial progenitor cells (Yamamoto, K. et al., Arterio. Thromb. Vasc. Biol., 24:192, 2004).

However, because the stem cells show the ability of differentiation into limited tissues, including differentiation into osteocytes or skeleton muscles by mesenchymal stem cells, differentiation into heart cells by heart stem cells, and differentiation into vascular endothelial cells by endothelial progenitor cells, it is difficult to define these cells as true multipotent stem cells. Also, in the above-cited prior literatures, an FBS-containing medium was used in a process of isolating stem cells, thus the number and kind of obtainable cells would be unavoidably limited.

Accordingly, the present inventors have cultured umbilical cord-derived blood using human serum or plasma as a medium, and as a result, found that the resulting adult cells have an excellent adhesion ability to plastics, differentiate into various tissues, such as osteogenic cells and nerve cells, and are useful as a cellular therapeutic agent for ischemic necrosis caused by occlusive arterial disease, thereby completing the present invention.

### DISCLOSURE OF THE INVENTION

It is a main object of the present invention to provide multipotent stem cells, which is adult stem cells derived from umbilical cord blood, and a method for preparing the same.

Another object of the present invention is to provide a cellular therapeutic agent which contains the adult stem cells as active ingredients for the treatment of ischemic necrosis and cardiovascular diseases caused by occlusive arterial disease.

To achieve the above objects, in one aspect, the present invention provides adult stem cells which are obtained by culturing umbilical cord-derived blood in a medium containing 5-20% of human serum or plasma and show the characteristics of:
(a) showing positive immunological responses to all of CD24, CD29, CD31, CD33, CD45, CD73 and CD49B, and negative immunological responses to CD34, CD51/61, CD62L, CD62P, CD90, CD133 and CD135;
(b) growing, being adhered to plastics and showing round-shaped or spindle-shaped morphological features; and
(c) having the ability to differentiate into the cells derived from mesoderm, endoderm and ectoderm.

In another aspect, the present invention provides a method for producing adult stem cells showing the characteristics (a) to (c), the method comprising the steps of: culturing umbilical cord-derived blood in a medium containing 5-20% of human serum or plasma; and recovering the adult stem cells.

In the present invention, the adult stem cells additionally show positive immunological responses to SH-2 and/or SH-3 and positive or negative immunological responses to CD44, CD105 and CD117. Also, the cells derived from mesoderm are osteogenic cells, nerve cells or endothelial cells.

In yet another aspect, the present invention provides a cellular therapeutic agent which contains the adult stem cells as active ingredients for the treatment of ischemic necrotic disease or cardiovascular diseases caused by occlusive arterial or veinous disease. The ischemic necrotic disease includes myocardiac infarction, cerebral infarction, avascular necrosis of hip joint, Buerger's disease, and gangrene of extremities resulting from diabetic complications.

The above and other objects, features and embodiments of the present invention will be more clearly understood from the following detailed description and the accompanying claims.

### BRIEF DESCRIPTION OF DRAWINGS

FIG. 1 illustrates photographs showing a comparison of cell adhesion between cells obtained by culturing umbilical cord blood in FBS and cells obtained by culturing umbilical cord blood in human plasma-containing medium. Left photographs in FIG 1 were taken at 200x magnification and right photographs were taken at 400x magnification.
FIGs. 2A and 2B show that multipotent stem cells derived from umbilical cord blood differentiated into osteogenic cells, and FIGs. 2C and 2D show the results of Von-Kossa staining.
FIG. 3 shows the binding between umbilical cord blood-derived multipotent stem cells and specific antigens. FIGs. 3A and 3B illustrate that the multipotent stem cells show positive responses to NSE (neuron-specific enolse), a neuron-specific antigen, and GFAP (glial fibrillary acidic protein), an astrocyte-specific antigen, respectively, and C and D show control groups.
FIG. 4 shows the immunological characteristics of umbilical cord blood-derived multipotent stem cells, measured by flow cytometry. A and E: control groups; B: CD34; C: CD45; D: SH-2; and F: SH-3.
FIG. 5 shows the results of PAS staining conducted to examine the expression or non-expression of antigens in the inventive adult stem cells.
FIG. 6 shows results for 30 days after vascular severance of a control mouse group with ischemic necrosis, in which a red circle shows a region where an amputation occurred.
FIG. 7 shows results for 30 days after the administration to a mouse group with ischemic necrosis, which was administered with the inventive stem cells immediately after vascular severance, in which a red circle shows a region where an amputation occurred.
FIG. 8 shows results for 31 days after the administration to a mouse group with ischemic necrosis, which was administered with the inventive stem cells one day after vascular severance, in which a red circle shows a region where an amputation occurred.
FIG. 9 is a graphic diagram showing symptom days and amputation days in mouse model with ischemic necrosis.
FIG. 10 is a graphic diagram showing the amputation rate of each group in mouse model with ischemic necrosis.
FIG. 11 illustrates X-ray photographs taken by angiography 30 days after vascular severance for each group of mouse model with ischemic necrosis.
FIG. 12 shows that human cells are traced with a human-specific probe using an in *situ* hybridization technique by sectioning femoral muscle tissues after the autopsy of a mouse model with ischemic necrosis.

### DETAILED DESCRIPTION OF THE INVENTION

The present invention relates to multipotent stem cells isolated from umbilical cord blood.

As used herein, the term "umbilical cord blood" is defined as blood collected from umbilical veins connecting the placenta with a fetus. The multipotent stem cells according to the present invention are preferably derived from human umbilical cord blood.

Although a method for isolating and purifying multipotent stem cells from umbilical cord blood is not specifically limited, the following method can be used. First, blood collected from umbilical cord is diluted with PBS at a given ratio to stir, and the stirred solution is separated on ficoll at a ratio of 10-15:20-30, and preferably 15:25. For this purpose, to 10-20 ml and preferably 15 ml of a ficoll solution, the above blood sample solution is spilled smoothly to cause layer separation, followed by centrifugation. Then, once three different layers are formed, a buffer coat of the middle layer is taken by a micropipette, washed 2-5 times with HBSS, and centrifuged to collect an umbilical cord blood-derived multipotent stem cell solution.

Pellets finally obtained by centrifugation are diluted in 1-5 ml of medium (MSCBM+MSCGM (Cambrex, USA) cell culture broth) containing 5-15% of autologous or allologous human serum or plasma and 10 ng/ml of basic fibroblast growth factor (bFGF)), and cultured on a flask. In the case of seeding into a 75-flask, about 10⁶-10⁸ cells are placed, to which 5-20% of autologous or allologous human serum and 10 ng/ml of basic fibroblast growth factor (bFGF) are added. The cells are cultured in a 5% CO₂ incubator at 37°C. The cells first seeded into the flask are transferred after about 1-2 days. The medium is replaced one time at about 3-7-day intervals.

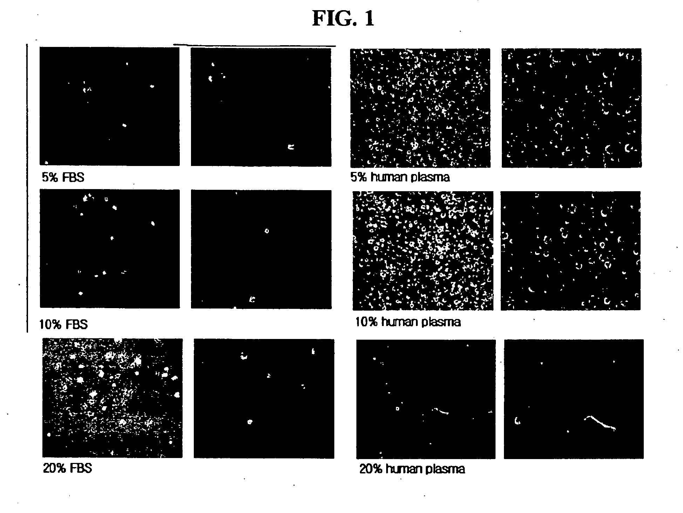

In the present invention, the umbilical cord blood-derived multipotent stem cell solution was cultured in a human serum or plasma-containing medium in place of the existing FBS. As a result, as shown in FIG. 1, the number of cells growing, being adhered to a culture dish was much larger in media containing 5% and 10% human plasma than in media containing 5% FBS and 10% FBS.

Methods of obtaining multipotent stem cells expressing the desired surface antigens from the umbilical cord blood-derived stem cell broth obtained above include a FACS method using a flow cytometer with sorting function *(*Int. Immunol., 10(3):275, 1998), a method using magnetic beads, and a panning method using an antibody specifically recognizing multipotent stem cells (J. Immunol., 141(8):2797, 1998). Also, methods for obtaining multipotent stem cells from a large amount of culture broth include a method where antibodies specifically recognizing molecules expressed on the surface of cells (hereinafter, referred to as "surface antigens") are used alone or in combination as columns.

Flow cytometry sorting methods may include a water drop charge method and a cell capture method. In any of these methods, an antibody specifically recognizing an antigen on the cell surface is fluorescently labeled, the intensity of fluorescence emitted from an antibody bonded with the molecule expressed on the surface of the cell is converted to an electric signal whereby the expressed amount of the antigen can be quantified. It is also possible to separate cells expressing a plurality of surface antigens by combination of fluorescence types used therefor. Examples of a fluorescence which can be used in this case include FITC (fluorescein isothiocyanate), PE (phycoerythrin), APC (allo-phycocyanin), TR (Texas Red), Cy 3, CyChrome, Red 613, Red 670, TRI-Color, Quantum Red, etc.

FACS methods using a flow cytometer include: a method where the above stem cell broth is collected, from which cells are isolated by, for example, centrifugation, and stained directly with antibodies; and a method where the cells are cultured and grown in a suitable medium and then stained with antibodies. The staining of cells is performed by mixing a primary antibody recognizing a surface antigen with a target cell sample and incubating the mixture on ice for 30 minutes to 1 hour. When the primary antibody is fluorescently labeled, the cells are isolated with a flow cytometer after washing. When the primary antibody is not fluorescently labeled, cells reacted with the primary antibody and a fluorescent labled secondary antibody having binding activity to the primary antibody are mixed after washing, and incubated on ice water for 30 minutes to 1 hour. After washing, the cells stained with the primary and secondary antibodies are isolated with a flow cytometer.

The method using magnetic beads allows mass isolation of cells expressing the target surface antigens. Although this method is lower in isolated cell purity than the above-described method using the flow cytometer, it can secure sufficient cell purity by repeated purification.

Surface antigens may include hematopoietic antigens, surface antigens of mesenchymal cells, and neuron-specific antigens of the nervous system. The hematopoietic antigens include CD34 and CD45, and the surface antigens of mesenchymal cells include SH-2 and SH-3, and the neuron-specific antigens of the nervous system include NSE, MAP2 and GFAP. The single or combined use of antibodies recognizing the above-described surface antigens allows the desired cells to be obtained.

In the present invention, the umbilical cord blood-derived multipotent stem cells obtained above were transplanted into a mouse model with ischemic necrosis, and as a result, it could be seen that, in cases administered with the inventive multipotent stem cells, the induction of necrotic symptoms was inhibited, and new blood vessels substituting for severed femoral arteries were produced.

Accordingly, the inventive multipotent stem cells can be used as a cellular therapeutic agent for the treatment of ischemic necrotic disease and cardiovascular diseases caused by occlusive arterial or vein disease. The cellular therapeutic agent for the treatment of ischemic necrotic disease preferably contains a high purity of cells having the differentiation ability into vascular cells, among the inventive multipotent stem cells.

The inventive multipotent stem cells are allowed to differentiate and proliferate *in vitro* depending on the size and site of ischemic necrosis, to obtain cells having the differentiation ability into a desired blood vessel, which can be used for the treatment of ischemic necrotic disease by transplantation them into ischemic necrotic sites. Also, the inventive multipotent stem cells may be transplanted directly into ischemic necrotic sites or systemically by intraveinousely injection.

The inventive multipotent stem cells show positive responses to monocyte-macrophage antigen CD45, and negative responses to hematopoietic lineage antigen CD34 thus are considered as stem cells which are in differentiation from hematopoietic cells into monocytes.

### Examples

Hereinafter, the present invention will be described in more detail by examples. It is to be understood, however, that these examples are for illustrative purpose only and are not construed to limit the scope of the present invention.

### Example 1: Isolation of multipotent stem cells from umbilical cord blood

Umbilical cord blood was collected from full-term and preterm newborns in Seoul National University Hospital and Samsung Cheil Hospital according to Institutional Review Board guidelines. 70-100 ml of a blood sample taken from the collected umbilical cord blood was diluted with PBS at a ratio of 1:1 followed by stirring. Then, the blood sample was separated on ficoll at a ratio of 15:25, in which the blood sample was spilled smoothly onto 15 ml of picoll solution to cause layer separation, followed by centrifugation at 2500 rpm for 20 minutes. After the centrifugation, three different layers were formed, and among them, a buffer coat of the middle layer was taken with a micropipette, washed three times with HBSS, followed by centrifugation at 1500 rpm for 15 minutes to obtain pellets (umbilical cord blood-derived multipotent stem cell broth).

### Example 2: Differentiation of umbilical cord blood-derived multipotent stem cells into osteogenic cells

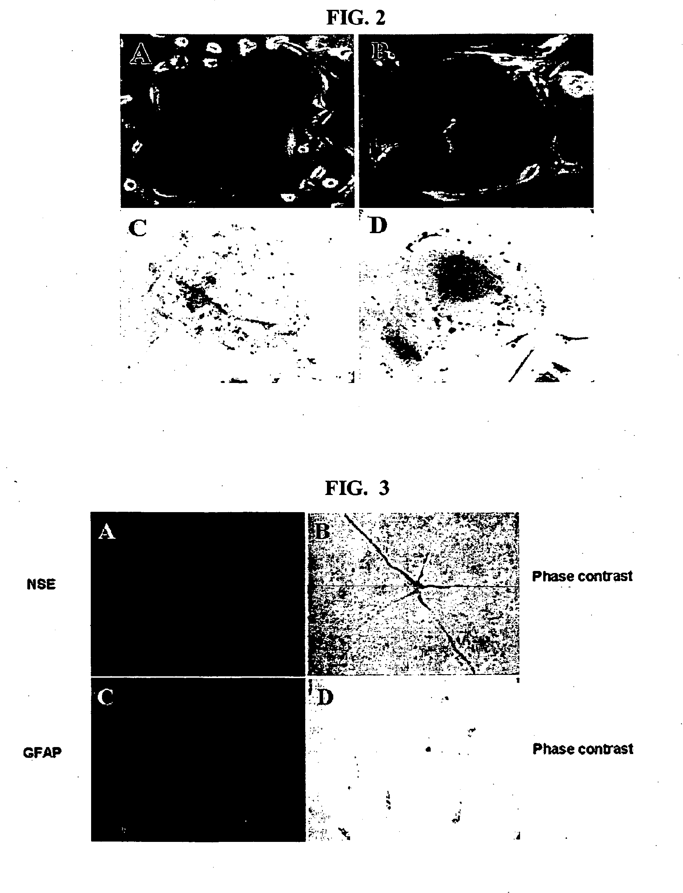

The umbilical cord blood-derived multipotent stem cell broth obtained in Example 1 was diluted in 1 ml of osteogenesis inducing medium (0.1 µmol/L dexamethasone (Sigma, USA), 0.05 mmol/L ascorbic acid-2-phosphate (Sigma, USA) and 10 mmol/L beta-glycophosphate (Sigma), 5-20% human serum or plasma) and counted for the number of cells. Then, the cells were cultured in a flask (5% CO₂; 37°C; medium replaced one time at 3-4-day intervals) to induce the differentiation of the multipotent stem cells into osteogenic cells. 14 days after the initiation of the culture, it was confirmed by Von-Kassa staining that the umbilical cord blood-derived multipotent stem cells differentiated into osteogenic cells (see FIG. 2).

### Example 3: Differentiation of umbilical cord blood-derived multipotent stem cells into nerve cells

The umbilical cord blood-derived multipotent stem cell broth obtained in Example 1 were diluted in 1 ml of nerve inducing medium (containing 10 ng/ml basic fibroblast growth (Roche, Switzerland), 10 ng/ml human epidermal growth factor (Roche, Switzerland) and 10ng/ml human neural growth factor (Invitrogen, USA) in 5-20% human serum or plasma) and counted for the number of cells. The cells were cultured on a flask in 5% CO₂ and at 37°C to induce differentiation from the multipotent stem cells into nerve cells. 14 days after the initiation of the culture, it was confirmed that the multipotent stem cells showed positive responses to NSE (neuron-specific enolase), a neuron-specific antigen, and GFAP (glial fibrillary acidic protein), an astrocyte-specific antigen. This suggests that the umbilical cord blood-derived multipotent stem cells differentiated into nerve cells (see FIG. 3).

### Example 4: Immunological characteristics of umbilical cord blood-derived multipotent stem cells

To examine the immunological characteristics of the umbilical cord blood-derived multipotent stem cells obtained in Example 1, the expression pattern of cell-surface antigens was analyzed. For this purpose, 2x10⁶-10⁷ of the cells cultured in Example 1 were washed with PBS solution and incubated with antibodies to the respective antigens at room temperature. The expression or non-expression of the antigens was analyzed with a flow cytometer. Also, PAS (periodic acid staining) was conducted.

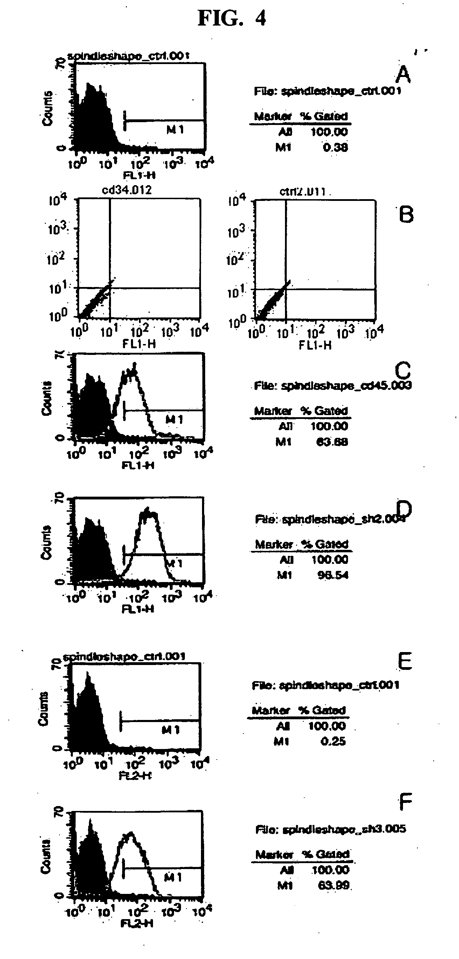

As a result, as shown in FIG. 4, the inventive umbilical cord blood-derived multipotent stem cells showed positive responses of 63.38%, 96.54% and 63.99% to CD45, SH-2 and SH-3, respectively, and a negative response of more than 90% to CD34. Also, immunophenotypes to other antigens were analyzed and as a result, the multipotent stem cells showed immunological characteristics of CD51/61-negative, CD62L-negative, CD62P-negative, CD133-negative, CD135-negative, CD90-negative, CD29-positive, CD44-positive or negative, CD49B-positive, CD105(SH-2)-positive or negative, and CD90-negative phenotypes.

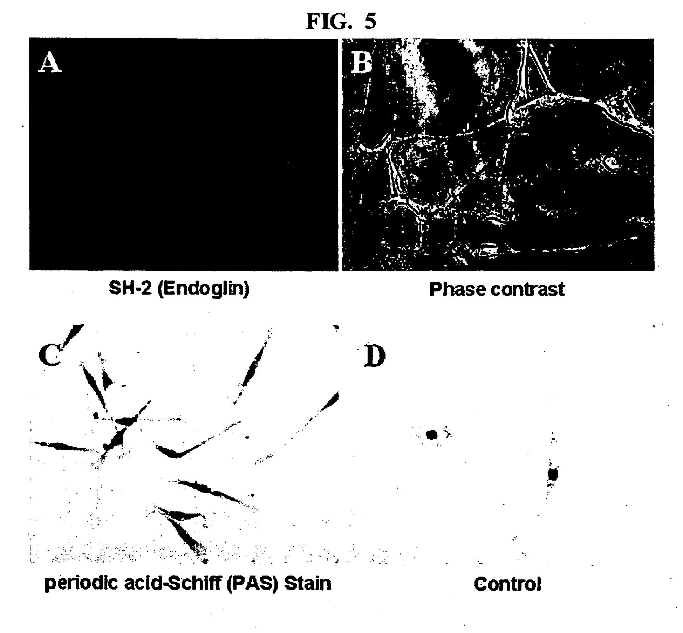

Meanwhile, as shown in FIG. 5, the multipotent stem cells showed positive response in PAS staining.

### Example 5: Therapeutic effect of multipotent stem cells on ischemic necrotic model

### (1) Preparation of test animals

Twenty 6-week-old female BALB/cANCrjBgi-nu mice were purchased from Orient Co., Ltd. and acclimated for one week. Then, among them, 18 animals were selected for use in test examples below. The selected animals were raised in an MI rack mounted with a HEPA filter at a temperature of 32 ± 3°C, a relative humidity of 60 ± 10%, ventilations of 10-12/hr, a lighting time of 12 hrs, and an illumination intensity of 250-2001ux while being allowed to freely take solid feed (Purina Co.) sterilized with steam under high pressure and drink water sterilized with steam under high pressure. During the acclimation and test periods, 7 animals per polycarbonate MI cage (26x42x18cm; manufactured by Myoung-jin Mechanical Co., Korea) were raised.

The raised mice were measured for their weights before test and randomly divided into the following three groups with no difference in their weights between groups: a control group consisting of 7 animals; a group consisting of 8 animals immediately administered with the multipotent stem cells; and a group consisting of 3 animals administered with the multipotent stem cells after one day (see Table 1).

**Table 1: Construction of test group**

| Groups | Sex | Numbers of animal | # |
|---|---|---|---|
| Control group | female | 7 | CF-1~CF-7 |
| Immediately administered group with the multipotent stem cells | female | 8 | MSCF-1~MSCF-8 |
| Media control | female | 3 | MSC-1F1-1~MSC-1F-3 |

According to the prior article (Nicholson, C.D. et al., Int. J. Sports Med., 13(1):60, 1992), the three animal groups were anesthetized by intra-abdominal injection of 1.2 mg ketamine, and then, the left femoral region of each individual was incised, and the femoral artery was severed and sutured to establish a mouse model with Buerger's disease.

The multipotent stem cells cultured in Example 1 were washed two times with PBS and treated with 0.25% trypsin, and the cells were collected and centrifuged to remove the supernatant. Then, after inactivating trypsin by washing with PBS, the cells were subjected to second centrifugation to collect 1.3x10⁶~10⁷ cells. The collected cells were suspended in 100 µl of sterilized physiological saline and administered into each femoral muscle of 8 mice (the group immediately administered with the multipotent stem cells). Also, culture was administered into each left femoral muscle of 3 mice (Media control).

### (2) Observation of symptom days and amputation days for ischemic necrotic model

During 30 days after the administration of the inventive multipotent stem cells to the mice with ischemic necrosis induced as described above, the femoral regions of the test animals were observed. Days showing symptom and days showing amputation were separately recorded, and before the initiation of test and at the end of test, the test animals were measured for their weights and subjected to autopsy after the completion of the test. For statistical analysis on the test animals' weights, and the like measured during the test, one-way ANOVA was carried out to examine the significance between the groups, and when the significance was acknowledged, Dunnett's t-test was carried out to examine the statistical significance between the control group and the test groups (p<0.05).

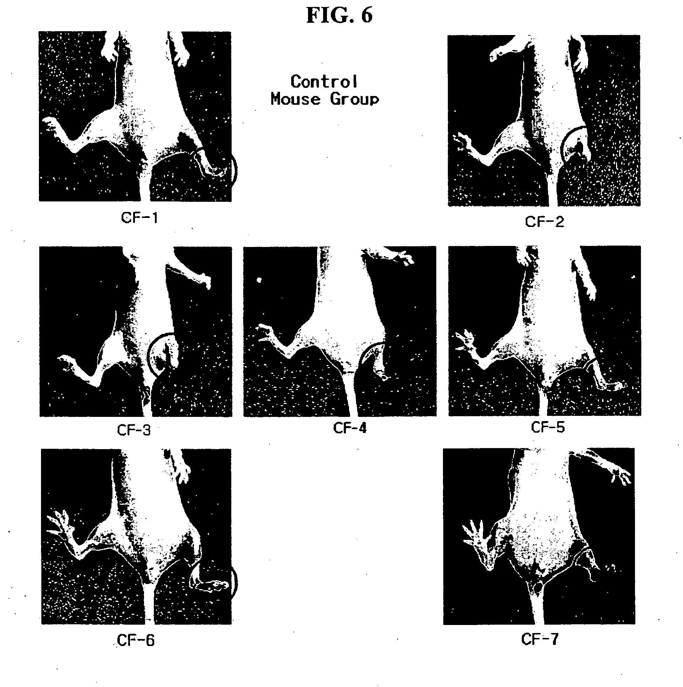

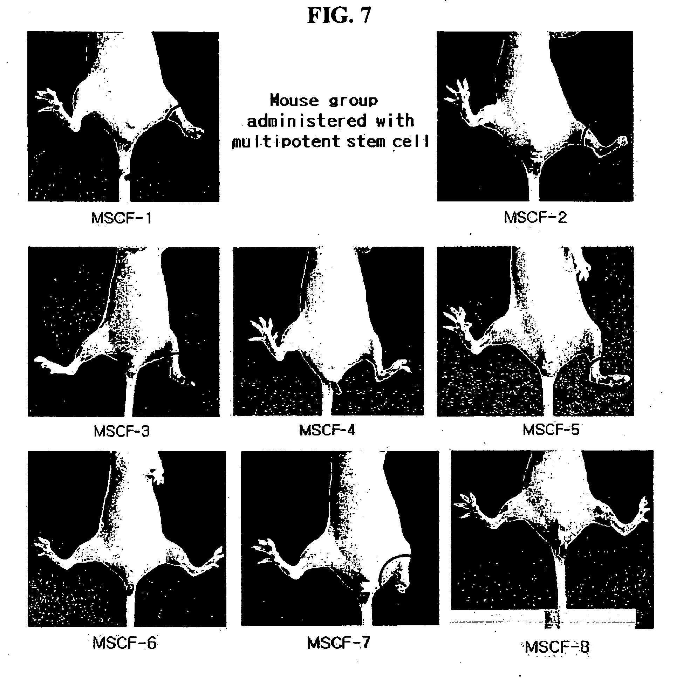

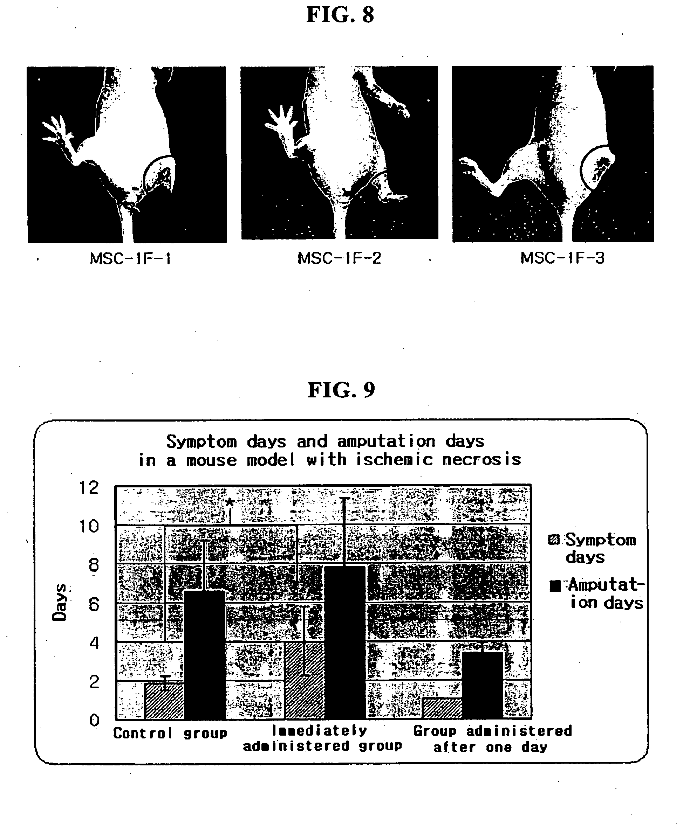

As a result, as shown in FIGS. 6 to 8, all the members of the control group untreated with the inventive umbilical cord blood-derived multipotent stem cells showed a cut phenomenon (observed in a red circle) 30 days after the severing of blood vessels (see FIG. 6). On the other hand, the group immediately administered with the multipotent stem cells showed an amputation in only five animals among a total of 8 animals, and individual Nos. 6 and 8 showed almost normal behavior patterns even if they had induced Buerger's disease, and individual No. 4 was prevented from being amputated by Buerger's disease (see FIG. 7). Meanwhile, the group administered with culture media showed the occurrence of amputation in all the three test animals, suggesting that this group has little or no therapeutic effect on the treatment of angiogenesis as compared to the group administered immediately with the multipotent stem cells (see FIG. 8).

Furthermore, on the basis of the day when the ischemic necrosis model was established, each individual was examined for days showing symptoms (feeling cold at severed vascular regions and showing a Raynaud's phenomenon) and for days showing the amputation of the left foot or toe (Table 2).

**Table 2**

| Groups | No. of animal | Weight before experiment (g)±SD | Weight after experiment (g)±SD | No. of amputated animal (amputation rate) | Symptom days ± standard deviation | Amputation days ± standard deviation |
|---|---|---|---|---|---|---|
| Control group | 7 | 20.0 ±0.89 | 20.2 ±1.07 | 7 (100%) | 1.86 ±0.38 | 6.57 ±2.64 |
| Immediately administered group with the multipotent stem cells | 8 | 20.0 ±0.74 | 20.4 ±0.55 | 5 (62.5%) | 4.00 ±1.77 | 7.80 ±3.56 |
| Media control | 3 | 20.1 ±1.27 | 20.4 ±1.78 | 3 (100%) | 1.00 ±0 | 3.33 ±0.58 |

As a result, as shown in Table 2, the group immediately administered with the multipotent stem cells showed a significance of less than 0.05 in the symptom days and the amputation days, and the remaining groups did not show significance. The weight of each individual was measured and calculated for the average and standard deviation of each group, and as a result, a significant change in their weights measured before and after the test was not observed.

Meanwhile, the symptom days and the amputation days were compared between the control group and the groups administered with the inventive umbilical cord blood-derived multipotent stem cells (FIG. 9). As a result, the group immediately administered with the multipotent stem cells showed a statistically significant change of less than 0.05 in the symptom days as compared to the control group. Although there was no significant change in the amputation days, the group immediately administered with the multipotent stem cells was longer in the amputation days than the control group and media control group, indicating that an amputation by vascular severance occurs slowly in the group immediately administered with the multipotent stem cells.

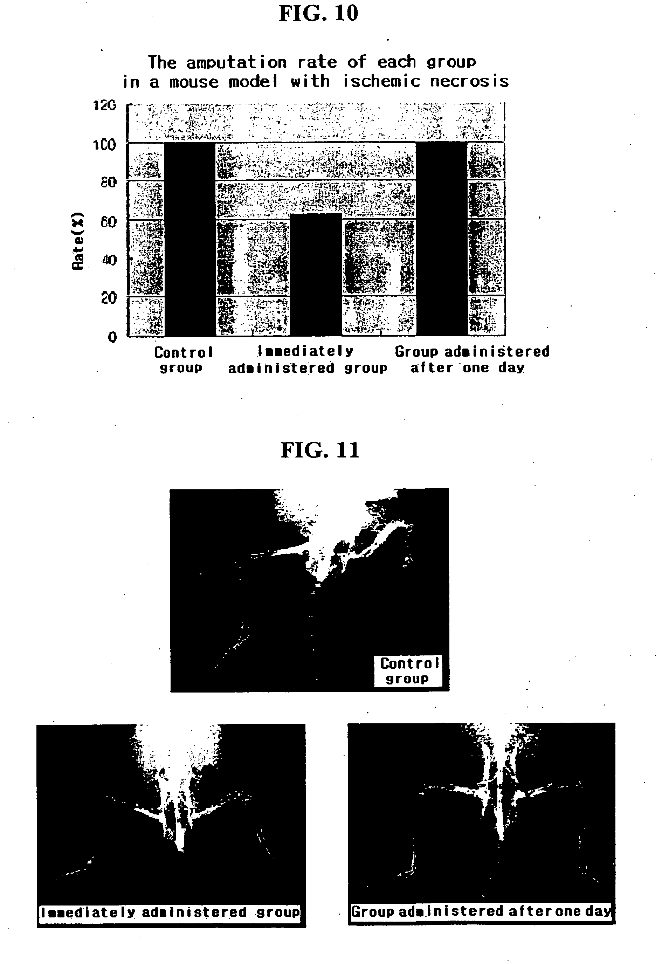

Amputation rate for each group with ischemic necrosis was examined, and as a result, as shown in FIG. 10, the control group and the group administered with the multipotent stem cells after one day showed an amputation rate of 100%, whereas the group administered immediately with the multipotent stem cells showed an amputation rate of 62.5%. These results suggest that the administration of the multipotent stem cells immediately after vascular severance allows an amputation to be reduced and also advantageously acts to lower an amputation line.

### (3) Angiography

Angiography is a vascular evaluation using X-ray and makes it possible to see blood vessels on X-rays by injecting a contrast agent (iodine-113) into the blood vessels of the hearts of mice. The presence or absence of abnormality in the blood vessels is examined on X-rays to be able to determine the name of disease, the location of lesions, or the progression of disease.

Before autopsy, each individual of the control group, the group immediately administered with the multipotent stem cells, and the group administered with the multipotent stem cells after one day, was anesthetized by intra-abdominal injection with ketamine (100 mg/kg), and administered with a contrast agent. After 2 minutes, each group was observed for femoral arteries and new blood vessels using X-rays. As a result, as shown in FIG. 11, new blood vessels was not observed in the control group and the group administered with the multipotent stem cells after one day, but was observed in the group immediately administered with the multipotent stem cells, where the amputation of the left lower limb did not occur.

### (4) In situ hybridization

Upon autopsy, the muscular blood of all the individuals of each group was removed by the collection of heart blood. Muscular tissues in the left and right femoral regions of each individual of the animal groups were fixed with a fixing solution consisting of a mixture of 4% paraformaldehyde phosphate solution and 1.5% sucrose solution. The fixed femoral tissues were left stand at 4°C until they settled in 30% sucrose phosphate solution. Then, each of the tissues was embedded in paraffin and finely sectioned with a microtome in a thickness of 5 µm. Then, the sections were reacted with prehybridization solution (50% formamide, 4XSSC, 50mM DDT, 4 x Denhart's solution, x TED, 100 µg/ml denatured salmon sperm DNA, 250 µg/ml yeast RNA) at 42°C for 1 hour. To the rssulting solution, DIG-labeled DNA (100 ng/ml) was added and reacted with the prehybridization solution for 24 hours so that a DIG labeled human specific DNA probe was bound to mRNA. The femoral tissues were washed with each of 2X, IX and 0.5X SSC solutions two times for 10 minutes each time, and fixed on a slide glass, followed by drying at room temperature for 2 hours.

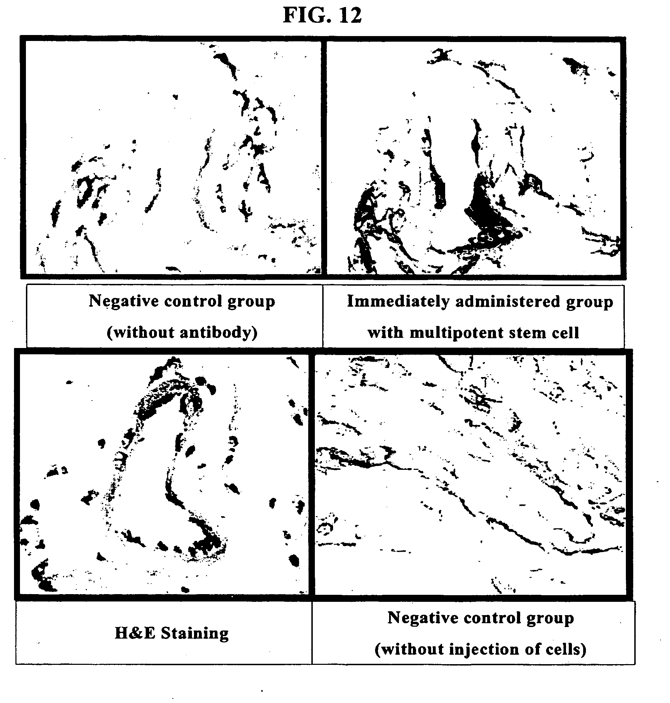

As a result, as could be seen in FIG. 12, the control group was not labeled with the human probe, but the individuals of the group administered with the multipotent stem cells were labeled with the human probe in endothelial cells.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

### INDUSTRIAL APPLICABILITY

As described in detail above, the present invention provides multipotent stem cells isolated from umbilical cord blood using human serum or plasma. The stem cells according to the present invention have the ability to differentiate into osteogenic cells, nerve cells, and the like, even if they are adult stem cells. Thus, the inventive stem cells will be useful as a cellular therapeutic agent for ischemic necrotic diseases caused by occlusive or ischemic arterial disease and cardiovascular diseases.

## Claims

1. Adult stem cells which are obtained by culturing umbilical cord-derived blood in a medium containing 5-20% of human serum or plasma and show the characteristics of:
(a) showing positive immunological responses to all of CD24, CD29, CD31, CD33, CD45, CD73 and CD49B, and negative immunological responses to CD34, CD51/61, CD62L, CD62P, CD90, CD133 and CD135;
(b) growing, being adhered to plastics and showing round-shaped or spindle-shaped morphological features; and
(c) having the ability to differentiate into the cells derived from mesoderm, endoderm and ectoderm.

2. The adult stem cells according to claim 1, which additionally show positive immunological responses to SH-2 and/or SH-3.

3. The adult stem cells according to claim 1, which show positive or negative immunological responses to CD44, CD105 and CD117.

4. The adult stem cells according to claim 1, wherein the cells derived from mesoderm are osteogenic cells, nerve cells or endothelial cells.

5. A method for producing adult stem cells showing the following characteristics:
(a) showing positive immunological responses to all of CD24, CD29, CD31, CD33, CD45, CD73 and CD49B, and negative immunological responses to CD34, CD51/61, CD62L, CD62P, CD90, CD133 and CD135;
(b) growing, being adhered to plastic and showing round-shaped or spindle-shaped morphological features; and
(c) having the ability to differentiate into the cells derived from mesoderm, endoderm and ectoderm,
the method comprising the steps of: culturing umbilical cord-derived blood in a medium containing 5-20% of human serum or plasma; and recovering the adult stem cells.

6. The method for producing adult stem cells according to claim 5, wherein the human serum or plasma is autologous or allologous.

7. A cellular therapeutic agent which contains the adult stem cells according to any one of claims 1 to 4 as active ingredients for the treatment of ischemic necrotic disease or cardiovascular diseases caused by occlusive arterial or veinous disease.

8. The cellular therapeutic agent according to claim 7, wherein the ischemic necrotic disease is selected from the group consisting of myocardiac infarction, cerebral infarction, avascular necrosis of hip joint, Buerger's disease, and gangrene of extremities resulting from diabetic complications.

## Patentansprüche

1. Adulte Stammzellen, die erhalten werden, indem aus Nabelschnur stammendes Blut in einem Medium, das 5-20% humanes Serum oder Plasma enthält kultiviert werden, und die die folgenden Charakteristika zeigen:
(a) zeigen positive immunologische Reaktionen auf alle von CD24, CD29, CD31, CD33, CD45, CD73 und CD49B und negative immunologische Reaktionen auf CD34, CD51/61, CD62L, CD62P, CD90, CD133 und CD135;
(b) wachsen, angeheftet an Kunststoffe, und zeigen rund geformte oder spindelförmige morphologische Merkmale und
(c) haben die Fähigkeit, in Zellen, abgeleitet von Mesoderm, Endoderm und Ektoderm, zu differenzieren.

2. Adulte Stammzellen gemäß Anspruch 1, die zusätzlich positive immunologische Reaktionen auf SH-2 und/oder SH-3 zeigen.

3. Adulte Stammzellen gemäß Anspruch 1, die positive oder negative immunologische Reaktionen auf CD44, CD105 und CD117 zeigen.

4. Adulte Stammzellen gemäß Anspruch 1, wobei die Zellen, die vom Mesoderm abgeleitet sind, osteogene Zellen, Nervenzellen oder Endothelialzellen sind.

5. Verfahren zum Herstellen von adulten Stammzellen, die die folgenden Charakteristika zeigen:
(a) zeigen positive immunologische Reaktionen auf alle von CD24, CD29, CD31, CD33, CD45, CD73 und CD49B und negative immunologische Reaktionen auf CD34, CD51/61, CD62L, CD62P, CD90, CD133 und CD135;
(b) wachsen, angeheftet an Kunststoff, und zeigen rund geformte oder spindelförmige morphologische Merkmale und
(c) haben die Fähigkeit, in Zellen, abgeleitet von Mesoderm, Endoderm und Ektoderm, zu differenzieren,
wobei das Verfahren die Schritte: Kultivieren von aus Nabelschnur stammendem Blut in einem Medium, das 5-20% humanes Serum oder Plasma enthält, und Gewinnen der adulten Stammzellen, umfasst.

6. Verfahren zur Herstellung von adulten Stammzellen gemäß Anspruch 5, wobei das humane Serum oder Plasma autolog oder allolog ist.

7. Zelltherapeutisches Mittel, das die adulten Stammzellen gemäß einem der Ansprüche 1 bis 4 als aktive Ingredienzien enthält, für die Behandlung von ischämischer Nekrose oder kardiovaskulären Erkrankungen, die durch arterielle oder venöse Verschlusskrankheit verursacht werden.

8. Zelltherapeutisches Mittel gemäß Anspruch 7, wobei die ischämische Nekrose ausgewählt ist aus der Gruppe, bestehend aus Myokardinfarkt, Hirninfarkt, avaskulärer Nekrose des Hüftgelenks, Bürger-Erkrankung und Gewebsnekrose von Extremitäten, resultierend aus diabetischen Komplikationen.

## Revendications

1. Cellules souches adultes obtenues par culture du sang dérivé du cordon umbilical dans un milieu contenant de 5 à 20 % de-sérum ou plasma humain, et montrant les caractéristiques suivantes:
a) elles montrent des réactions immunologiques positives contre tous de CD24, CD29, CD31, CD33, CD45, CD73 et CD49B, et des réactions immunologiques négatives contre CD34, CD51/61, CD62L, CD62P, CD90, CD133 et CD135;
b) elles croissent en étant adhérées à des matières plastiques et montrent des caractéristiques morphologiques de forme ronde ou de forme de fuseau; et
c) elles possèdent la capacité de se différencier en cellules dérivées du mésoderme, de l'endoderme et de l'ectoderme.

2. Cellules souches adultes selon la revendication 1, qui montrent en plus des réactions immunologiques positives contre SH-2 et/ou SH-3.

3. Cellules souches adultes selon la revendication 1, qui montrent des réactions immunologiques positives ou négatives contre CD44, CD105 et CD117.

4. Cellules souches adultes selon la revendication 1 **caractérisées en ce que** les cellules dérivées du mésoderme sont des cellules ostéogéniques, des cellules nerveuses ou des cellules endothéliales.

5. Procédé de production de cellules souches adultes montrant les caractéristiques suivantes:
a) elles montrent des réactions immunologiques positives contre tous de CD24, CD29, CD31, CD33, CD45, CD73 et CD49B, et des réactions immunologiques négatives contre CD34, CD51/61, CD62L, CD62P, CD90, CD133 et CD135;
b) elles croissent en étant adhérées à une matière plastique et montrent des caractéristiques morphologiques de forme ronde ou de forme de fuseau; et
c) elles possèdent la capacité de se différencier en cellules dérivées du mésoderme, de l'endoderme et de l'ectoderme,
ladite méthode comprenant les étapes suivantes: cultiver du sang dérivé du cordon umbilical dans un milieu contenant de 5 à 20 % de sérum ou plasma humain; et récupérer les cellules souches adultes.

6. Procédé de production de cellules souches adultes selon la revendication 5, **caractérisé en ce que** le sérum ou plasma humain est autologue ou allologue.

7. Agent thérapeutique cellulaire contenant les cellules souches adultes selon l'une quelconque des revendications 1 à 4 comme ingrédients actifs pour le traitement d'une maladie nécrotique ischémique ou des maladies cardiovasculaires causée(s) par une maladie occlusive artérielle ou veineuse.

8. Agent thérapeutique cellulaire selon la revendication 7 **caractérisé en ce que** la maladie nécrotique ischémique est sélectionnée parmi le groupe composé de l'infarctus de myocarde, l'infarctus cérébral, la nécrose avasculaire de l'articulation de la hanche, la maladie de Buerger et le gangrène d'extrémités résultant de complications diabétiques.
